# EUROPEAN PATENT APPLICATION

(11) **EP 4 413 983 A1**
(43) Date of publication of application: **14.08.2024**
(21) Application number: 22878604.2
(22) Date of filing: 07.10.2022
(51) Int. Cl.: A61K 31/36, A23L 33/105, A61P 1/04

(54) **ENTERITIS-SUPPRESSING COMPOSITION**

(30) Priority: 08.10.2021 JP 2021166013
(71) Applicant: Kobe Gakuin Educational Foundation, Kobe-shi, Hyogo 650-8586 (JP); Kiyomoto Iron & Machinery Works Co. Ltd., Nobeoka-shi, Miyazaki 889-0595 (JP)
(72) Inventor: OHIRA, Hideo, Kobe-shi, Hyogo 651-2180 (JP); KIYOMOTO, Kunio, Kobe-shi, Hyogo 651-2180 (JP)
(74) Representative: Held, Stephan
(86) International application number: PCT/JP2022/037541
(87) International publication number: WO 2023/058737

(57) **Abstract**

The problem to be solved by the invention is to provide a composition for suppressing enteritis. The problem is solved by a composition for suppressing enteritis, comprising at least one member selected from the group consisting of sesaminol and a metabolite thereof.

## Description

### Technical Field

The present disclosure relates to, for example, a composition for suppressing enteritis.

### Background Art

Enteritis is the general term for inflammation of intestinal mucosa of the duodenum, small intestine, large intestine, etc. Colitis is the general term for mucosal inflammation of the colon and rectum. It has been reported that colitis can be caused by, for example, infections with bacteria, viruses, etc.; a recent report indicates that colitis can be caused by alcohol consumption etc. Additionally, chronic colitis has been reported to be a risk factor for the development of colorectal cancer.

Recommended means for treating ulcerative colitis currently vary depending on the severity. According to the Diagnostic Criteria and Treatment Guidelines for Ulcerative Colitis and Crohn's Disease (Non-Patent Literature (NPL) 1) in a scientific research program report (Hisamatsu Group) by the Ministry of Health, Labour and Welfare in Japan, for example, the use of 5-aminosalicylic acid is recommended for mild conditions and remission maintenance treatment, the use of steroid drugs and surgical operations are recommended for severe conditions, and the use of high-dose intravenous steroids and immunosuppressive agents is recommended for fulminant conditions.

The cause of the onset of ulcerative colitis still remains unknown, and ulcerative colitis continues to be designated as a target disease of the project for countermeasures against intractable diseases of the Ministry of Health, Labour and Welfare in Japan (designated intractable disease No. 97, as of August 2021) (NPL 2). However, many types of colitis, including ulcerative colitis, have the potential for prevention of the onset and for delay of the progress through daily diet and through effective use of functional health foods, which can be important methods for attempting health improvement for all people from children to the elderly (NPL 3).

It is widely known that gut microbiota plays an important role in the onset and progression of inflammatory bowel diseases (IBDs) (NPL 4). Given this, many techniques for restoring the intestinal environment back to the normal state have been attempted to prevent colitis. One example is a functional health food containing *Bifidobacterium*, which regulates the dysbiosis of the configuration of gut microbiota (NPL 5 and Patent Literature (PTL) 1). Lentinan, which is a β-glucan contained in shiitake mushrooms (*Lentinula edodes*), has been reported to show an effect of suppressing colitis through inhibition of the expression of interleukin-8 (IL8) (NPL 6), and has attracted attention as a functional food ingredient.

Sesaminol is known to be present in sesame seeds. Sesaminol has also been reported to have an antioxidant function (NPL 7). It has recently been suggested that sesaminol has not only an antioxidant function but also a wide variety of physiological actions, such as Parkinson's disease prevention and cognitive impairment prevention (NPL 8, NPL 9, and NPL 10). However, there have so far been no reports on the action of sesaminol for suppressing enteritis.

### Citation List

### Patent Literature

PTL 1: JP2012-149032A

### Non-patent Literature

NPL 1: Health Labour Sciences Research Grant for Project for Research on Rare and Intractable Diseases, "Research on intractable intestinal inflammation" (Hisamatsu Group), Shared Research Report 2020, the Diagnostic Criteria and Treatment Guidelines for Ulcerative Colitis and Crohn's Disease, March 31, 2021
NPL 2: Designated Intractable Diseases (Notification Nos. 1 to 110), effective January 1, 2015, Notification No. 97: Ulcerative Colitis (https://www.mhlw.go.jp/stf/seisakunitsuite/bunya/0000062437.html )
NPL 3: Ministerial Notification No. 430 of the Ministry of Health, Labour and Welfare, Health Promotion Act (Act No. 103 of 2002), Article 7(1), Basic policy for comprehensively advancing the people's improvement of their health, 2. Prevention of onset and progression of lifestyle-related diseases (prevention of NCD)
NPL 4: II. Pathology and Pathophysiology of Inflammatory Bowel Diseases, 3. Role of gut microbiota, the Journal of the Japanese Society of Internal Medicine, Vol. 98, No. 1, January 10, 2009
NPL 5: Clinical Utility of Bifidobacteria-containing Capsules Designed to Dissolve in the Colon, Journal of Intestinal Microbiology, 17: 67-79, 2003
NPL 6: Nishitani Y, Zhang L, Yoshida M, Azuma T, Kanazawa K, Hashimoto T, et al. (2013), Intestinal Anti-Inflammatory Activity of Lentinan: Influence on IL-8 and TNFR1 Expression in Intestinal Epithelial Cells. PLoS ONE 8(4): e62441. https://doi.org/10.1371/journal.pone.0062441
NPL 7: Mebeaselassie Andargie, Maria Vinas, Anna Rathgeb et al., Lignans of Sesame (Sesamum indicum L.): A Comprehensive Review. Molecules. 2021; 26(4): 883. doi: 10.3390/molecules26040883.
NPL 8: M H Kang, M Naito, K Sakai, K Uchida, T Osawa, Mode of action of sesame lignans in protecting low-density lipoprotein against oxidative damage in vitro. Life Sci. 2000; 66: 161-71. doi: 10.1016/s0024-3205(99)00574-3.
NPL 9: Haruka Kaji, Isao Matsui-Yuasa, Kayo Matsumoto, Ayano Omura, Kunio Kiyomoto, Akiko Kojima-Yuasa, Sesaminol prevents Parkinson's disease by activating the Nrf2-ARE signaling pathway. Heliyon, 2020; 6: e05342. doi: 10.1016/j.heliyon.2020.e05342.
NPL 10: Min Young Um, Ji Yun Ahn, Suna Kim, Mi Kyung Kim, Tae Youl Ha, et al., Sesaminol glucosides protect beta-amyloid peptide-induced cognitive deficits in mice. Biol Pharm Bull. 2009; 32: 1516-20. https://doi.org/10.1248/bpb.32.1516.
NPL 11: Mika Mochizuki, Yoshikazu Tsuchie, Yoshimasa Nakamura, Toshihiko Osawa, Identification and characterization of sesaminol metabolites in the liver, J Agric Food Chem 2009; 57: 10429-34. https://doi.org/10.1021/jf901939m.
NPL 12: Kuo-Ching Jan, Kuo-Lung Ku, Yan-Hwa Chu, Lucy Sun Hwang, Chi-Tang Ho, Intestinal distribution and excretion of sesaminol and its tetrahydrofuranoid metabolites in rats, J Agric Food Chem 2011; 59: 3078-86. https://doi.org/10.1021/jf105012v.
NPL 13: Ohira H, Tsuruya A, Oikawa D, Nakagawa W, Mamoto R, Hattori M, et al. (2021) Alteration of oxidative-stress and related marker levels in mouse colonic tissues and fecal microbiota structures with chronic ethanol administration: Implications for the pathogenesis of ethanol-related colorectal cancer. PLoS ONE 16(2): e0246580. https://doi.org/10.1371/journal.pone.0246580

### Summary of Invention

### Technical Problem

An object of the present invention is to provide a novel composition for suppressing enteritis.

### Solution to Problem

As a result of extensive research, the present inventors found that sesaminol has an effect of suppressing enteritis. Based on this finding, the present inventors conducted further research and consequently developed a novel composition for suppressing enteritis, comprising at least one member selected from the group consisting of sesaminol and a metabolite thereof.

Specifically, the present invention encompasses the following embodiments.
Item 1. A composition for suppressing enteritis, comprising at least one member selected from the group consisting of sesaminol and a metabolite thereof.
Item 2. The composition for suppressing enteritis according to Item 1, wherein the enteritis is chronic enteritis.
Item 3. The composition for suppressing enteritis according to Item 1 or 2, for use in suppressing leaky gut syndrome.
Item 4. The composition for suppressing enteritis according to any one of Items 1 to 3, which is an oral composition.
Item 5. The composition for suppressing enteritis according to any one of Items 1 to 4, which is a food composition.
Item 6. The composition for suppressing enteritis according to any one of Items 1 to 5, wherein the metabolite is a sesaminol metabolite in the digestive tract.
Item 7. A composition for suppressing intestinal tissue damage, comprising at least one member selected from the group consisting of sesaminol and a metabolite thereof.
Item 8. A composition for protecting intestinal mucosa, comprising at least one member selected from the group consisting of sesaminol and a metabolite thereof.
Item 9. A composition for suppressing oxidative stress in intestinal tissue, comprising at least one member selected from the group consisting of sesaminol and a metabolite thereof.
Item 10. A composition for suppressing leaky gut syndrome, comprising at least one member selected from the group consisting of sesaminol and a metabolite thereof.
Item 11. A composition for improving gut microbiota, comprising at least one member selected from the group consisting of sesaminol and a metabolite thereof.

The present invention also encompasses the following embodiments.
Item 1A. A method for suppressing enteritis, comprising administering or feeding at least one member selected from the group consisting of sesaminol and a metabolite thereof to a subject in need of suppressing enteritis.
Item 1B. A composition comprising at least one member selected from the group consisting of sesaminol and a metabolite thereof, for use as a composition for suppressing enteritis.
Item 1C. Use of a composition comprising at least one member selected from the group consisting of sesaminol and a metabolite thereof, in the production of a composition for suppressing enteritis.
Item 1D. Use of a composition comprising at least one member selected from the group consisting of sesaminol and a metabolite thereof, as a composition for suppressing enteritis.
Item 7A. A method for suppressing intestinal tissue damage, comprising administering or feeding at least one member selected from the group consisting of sesaminol and a metabolite thereof to a subject in need of suppressing intestinal tissue damage.
Item 7B. A composition comprising at least one member selected from the group consisting of sesaminol and a metabolite thereof, for use as a composition for suppressing intestinal tissue damage.
Item 7C. Use of a composition comprising at least one member selected from the group consisting of sesaminol and a metabolite thereof, in the production of a composition for suppressing intestinal tissue damage.
Item 7D. Use of a composition comprising at least one member selected from the group consisting of sesaminol and a metabolite thereof, as a composition for suppressing intestinal tissue damage.
Item 8A. A method for protecting intestinal mucosa, comprising administering or feeding at least one member selected from the group consisting of sesaminol and a metabolite thereof to a subject in need of protecting intestinal mucosa.
Item 8B. A composition comprising at least one member selected from the group consisting of sesaminol and a metabolite thereof, for use as a composition for protecting intestinal mucosa.
Item 8C. Use of a composition comprising at least one member selected from the group consisting of sesaminol and a metabolite thereof, in the production of a composition for protecting intestinal mucosa.
Item 8D. Use of a composition comprising at least one member selected from the group consisting of sesaminol and a metabolite thereof, as a composition for protecting intestinal mucosa.
Item 9A. A method for suppressing oxidative stress in intestinal tissue, comprising administering or feeding at least one member selected from the group consisting of sesaminol and a metabolite thereof to a subject in need of suppressing oxidative stress in intestinal tissue.
Item 9B. A composition comprising at least one member selected from the group consisting of sesaminol and a metabolite thereof, for use as a composition for suppressing oxidative stress in intestinal tissue.
Item 9C. Use of a composition comprising at least one member selected from the group consisting of sesaminol and a metabolite thereof, in the production of a composition for suppressing oxidative stress in intestinal tissue.
Item 9D. Use of a composition comprising at least one member selected from the group consisting of sesaminol and a metabolite thereof, as a composition for suppressing oxidative stress in intestinal tissue.
Item 10A. A method of suppressing leaky gut syndrome, comprising administering or feeding at least one member selected from the group consisting of sesaminol and a metabolite thereof to a subject in need of suppressing leaky gut syndrome.
Item 10B. A composition comprising at least one member selected from the group consisting of sesaminol and a metabolite thereof, for use as a composition for suppressing leaky gut syndrome.
Item 10C. Use of a composition comprising at least one member selected from the group consisting of sesaminol and a metabolite thereof, in the production of a composition for suppressing leaky gut syndrome.
Item 10D. Use of a composition comprising at least one member selected from the group consisting of sesaminol and a metabolite thereof, as a composition for suppressing leaky gut syndrome.
Item 11A. A method for improving gut microbiota, comprising administering or feeding at least one member selected from the group consisting of sesaminol and a metabolite thereof to a subject in need of improving gut microbiota.
Item 11B. A composition comprising at least one member selected from the group consisting of sesaminol and a metabolite thereof, for use as a composition for improving gut microbiota.
Item 11C. Use of a composition comprising at least one member selected from the group consisting of sesaminol and a metabolite thereof, in the production of a composition for improving gut microbiota.
Item 11D. Use of a composition comprising at least one member selected from the group consisting of sesaminol and a metabolite thereof, as a composition for improving gut microbiota.

### Advantageous Effects of Invention

The present invention can provide a composition for suppressing enteritis, comprising at least one member selected from the group consisting of sesaminol and a metabolite thereof. The use of this composition can enable prevention, treatment, etc. of enteritis.

### Brief Description of Drawings

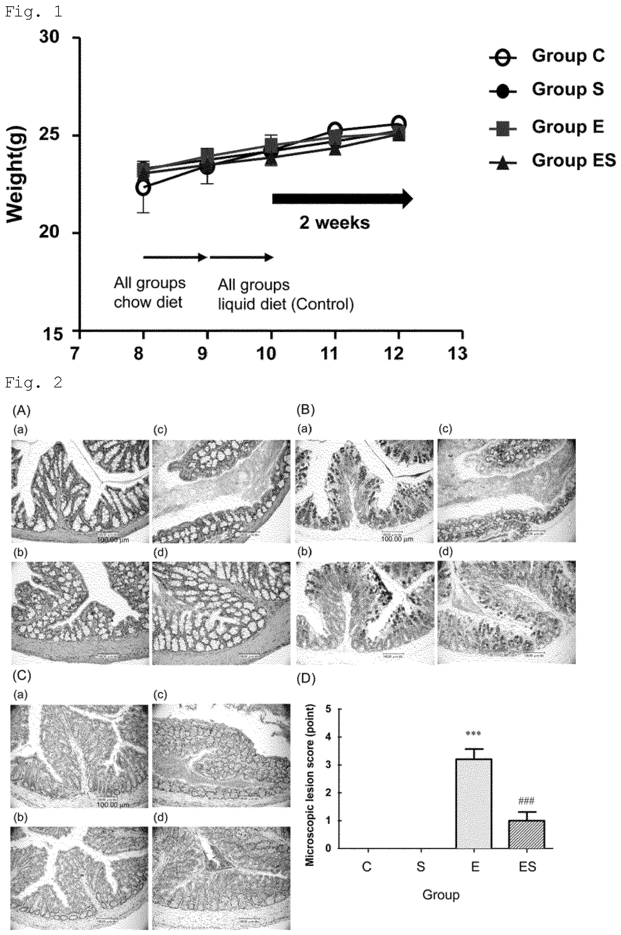

Fig. 1 shows changes in the body weight of the mice in group C, group S, group E, and group ES during the test period (2 weeks). In the graph, the white circle (∘) represents group C, the black circle (**●**) represents group S, the black square (**■**) represents group E, and the black triangle (▲) represents group ES. The data show the mean ±SD of each group (n=8).
Fig. 2 shows the results of microscopic evaluation of the effect of oral administration of sesaminol on ethanol-induced lesion in mouse colon. The colon tissue pieces of the mice 15 days after the start of the test (12 weeks old) were observed under a microscope. (A): HE-stained images, (B): TB-stained images, and (C): Immunostained images using anti-MUC2 antibody. (a): Group C, (b): Group S, (c): Group E, (d): Group ES, and (D): Microscopic lesion scores. The scores represent the mean ±SD (n=5). ***: p-value < 0.001 between groups C and E, ^{###}: p-value < 0.001 between groups E and ES.

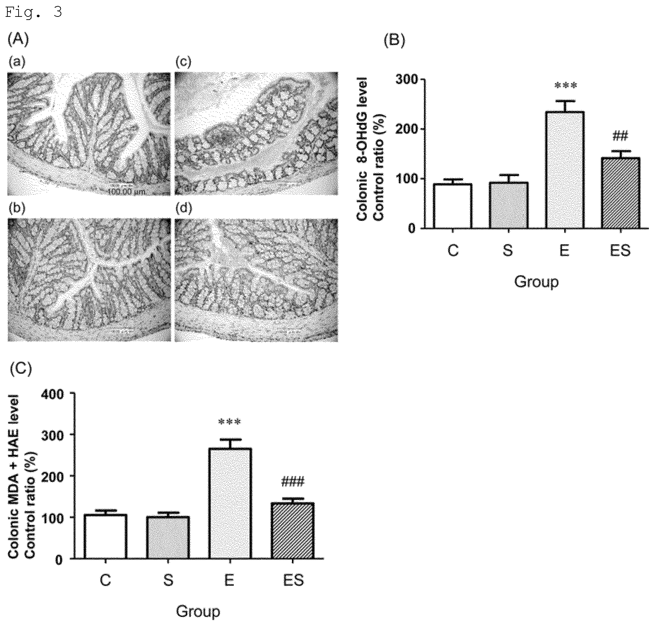

Fig. 3 shows the effect of oral administration of sesaminol on oxidative stress marker (8-OHdG and MDA+HAE) levels induced by chronic ethanol consumption in mouse colon. (A): Microscopic images of colon tissue pieces immunohistochemically stained with 8-OHdG. (a): Group C, (b): Group S, (c): Group E, and (d): Group ES. (B): Relative 8-OHdG level in each group obtained by detecting the 8-OHdG level in each colon tissue extract using ELISA and taking the 8-OHdG level in group C as 100%. (C): Relative MDA+HAE level in each group obtained by measuring the MDA+HAE level in each colon tissue extract and taking the MDA+HAE level in group C as 100%. ***: p-value < 0.001 between groups C and E, ^{##}: p-value < 0.01 between groups C and ES, ^{###}: p-value < 0.001 between groups E and ES.

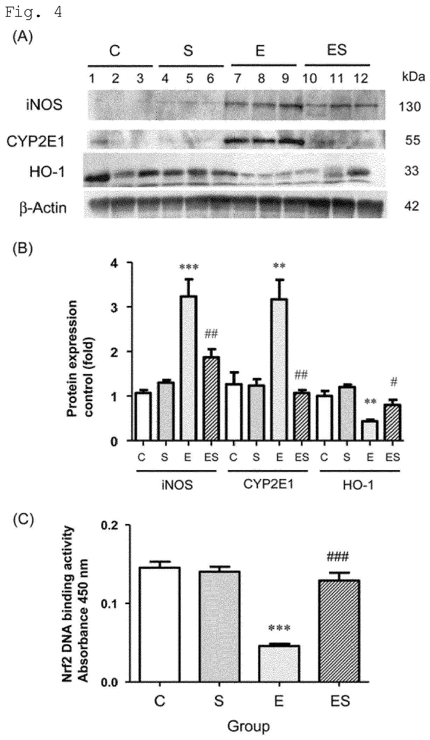

Fig. 4 shows the effect of oral administration of sesaminol on the levels of ethanol-induced oxidative stress proteins (iNOS, CYP2E1, and HO-1) and transcription factor protein (Nrf2) in mouse colon tissue. (A): Results of western blotting analysis of the expression levels of iNOS, CYP2E1, HO-1, and β-actin in each colon tissue extract of the mice in groups C, S, E, and ES. Lanes 1-3: group C, lanes 4-6: group S, lanes 7-9: group E, and lanes 10-12: group ES. (B): Expression levels of proteins of iNOS, CYP2E1, and HO-1 analyzed by WB. The band intensities were standardized by β-actin. The relative band intensity of each group is shown by taking the band intensity of group C as 1.0 (fold). The data show the mean ±SD (n=3). (C): Results of Nerf2 activation levels in colon tissue extracts examined by ELISA. The data show the mean ±SD (n=3). **: p-value < 0.01 between groups C and E, ***: p-value < 0.001 between groups C and E, ^{#}: p-value < 0.05 between groups E and ES, ^{##}: p-value < 0.01 between groups E and ES, ^{###}: p-value < 0.001 between groups E and ES.

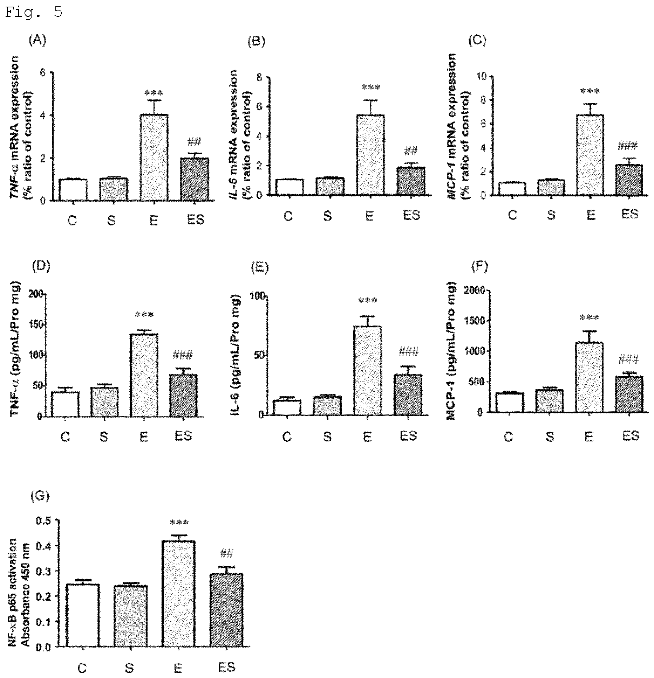

Fig. 5 shows relative mRNA levels of (A) TNF-α, (B) IL-6, and (C) MCP-1 analyzed by qRT-PCR with the mRNA level of each gene in group C taken as 1.0. The protein levels of (D) TNF-α, (E) IL-6, and (F) MCP-1 were measured by ELISA. The activation level of (G) NF-κB p65 in each colon tissue extract was measured by ELISA, and the relative NF-κB p65 activation levels are expressed as absorbance at 450 nm. The data show the mean ±SD (n=5). ***: p-value < 0.001 between groups C and E, ^{##}: p-value < 0.01 between groups E and ES, ^{###}: p-value < 0.001 between groups E and ES.

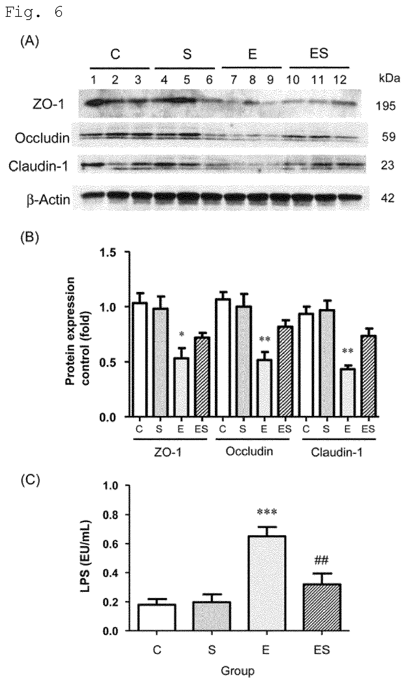

Fig. 6 shows the effect of oral administration of sesaminol on the expression levels of intestinal TJ proteins (ZO-1, occludin, and claudin-1) and the plasma LPS level. (A): Results of western blotting analysis of the expression levels of ZO-1, occludin, claudin-1, and β-actin in each colon tissue extract of the mice in group C (lanes 1-3), group S (lanes 4-6), group E (lanes 7-9), and group ES (lanes 10-12). (B): Relative band intensity of each group obtained by standardizing the band intensities in (A) with β-actin and taking the band intensity of group C as 1.0 (fold). The data show the mean ±SD (n=3). (C) shows the plasma LPS concentration of each group. The data show the mean ±SD (n=5). *: p-value < 0.05 between groups C and E, **: p-value < 0.01 between groups C and E, ***: p-value < 0.001 between groups C and E, ^{##}: p-value < 0.01 between groups E and ES.

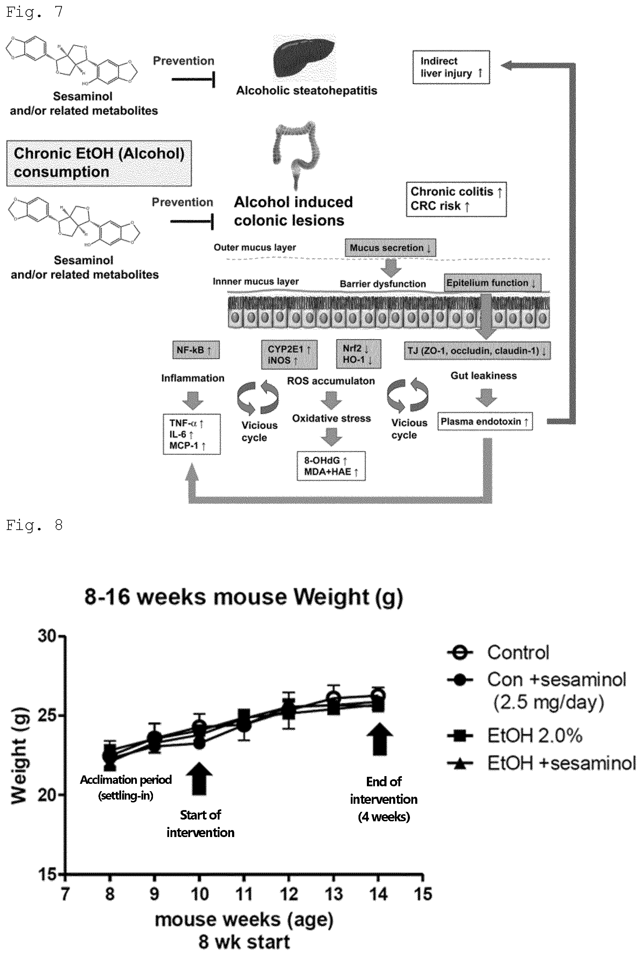

Fig. 7 is a schematic diagram of the present invention.
Fig. 8 shows changes in the body weight of the mice in group C, group S, group E, and group ES during the test period (4 weeks). In the graph, the white circle (∘) represents group C, the black circle (**●**) represents group S, the black square (**■**) represents group E, and the black triangle (▲) represents group ES. The data show the mean ±SD of each group (n=8).

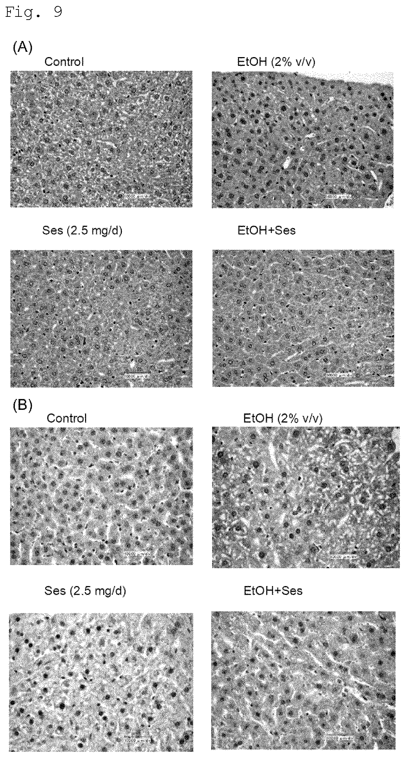

Fig. 9 shows the results of microscopic evaluation of ethanol-induced alcoholic steatohepatitis in mouse liver sections and the effect of oral administration of sesaminol. The liver sections of the mice 4 weeks after the start of the test (14 weeks old) were observed under a microscope. (A): HE-stained images and (B): Oil Red O-stained images. (a): Group C, (b): Group S, (c): Group E, and (d): Group ES.

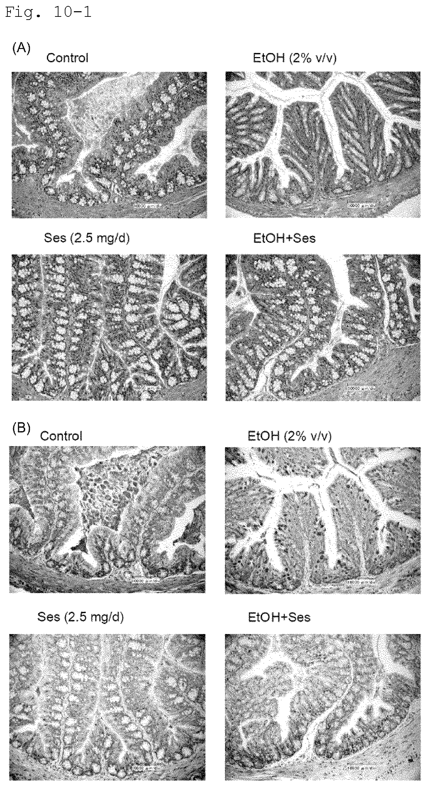

Fig. 10-1 shows the results of microscopic evaluation of the effect of oral administration of sesaminol on ethanol-induced lesions in mouse colon. The colon tissue pieces of the mice 4 weeks after the start of the test (14 weeks old) was observed under a microscope. (A): HE-stained images and (B) TB-stained images. (a): Group C, (b): Group S, (c): Group E, and (d): Group ES.

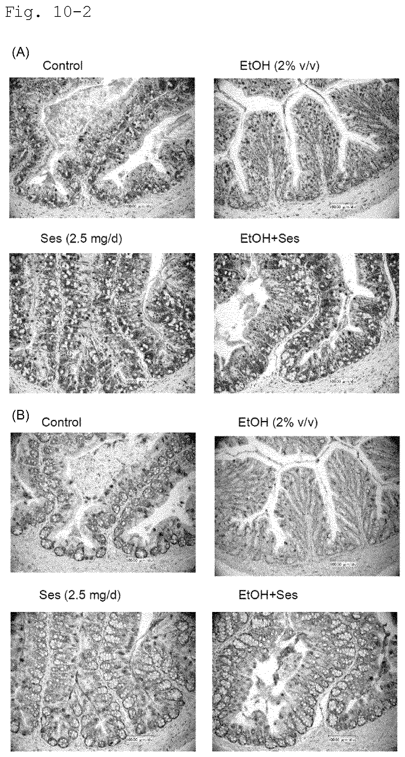

Fig. 10-2 shows the results of microscopic evaluation of the effect of oral administration of sesaminol on ethanol-induced lesions in mouse colon. The colon tissue pieces of the mice 4 weeks after the start of the test (14 weeks old) was observed under a microscope. (C): PAS-stained images and (D) immunostained images using anti-MUC2 antibody. (a): Group C, (b): Group S, (c): Group E, and (d): Group ES.

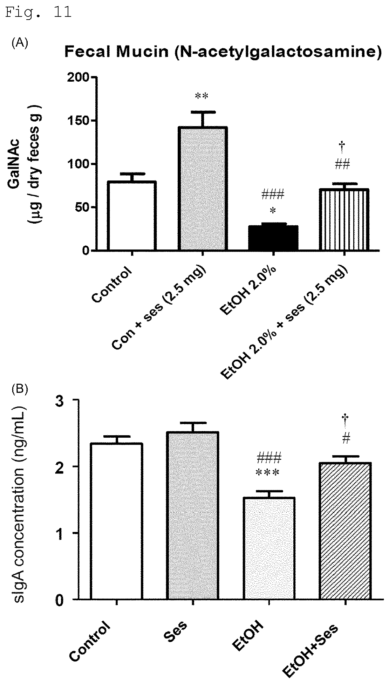

Fig. 11 shows the effect of oral administration of sesaminol on the amount of mucus secretion in mouse colon. (A): Measurement results of the amount of fecal mucin of the mice. The data show the mean ±SD (n=8). *: p-value < 0.05 between groups C and E, **: p-value < 0.01 between groups C and S, ^{##}: p-value < 0.01 between groups S and ES, ^{###}: p-value < 0.01 between groups S and E, and ^{†}: p-value < 0.05 between groups E and ES. (B): Measurement results of the amount of secretory IgA in colon tissue of the mice. The data show the mean ±SD (n=5). ***: p-value < 0.001 between groups C and E, ^{#}: p-value < 0.05 between groups S and ES, ^{###}: p-value < 0.01 between groups S and E, and ^{†}: p-value < 0.05 between groups E and ES.

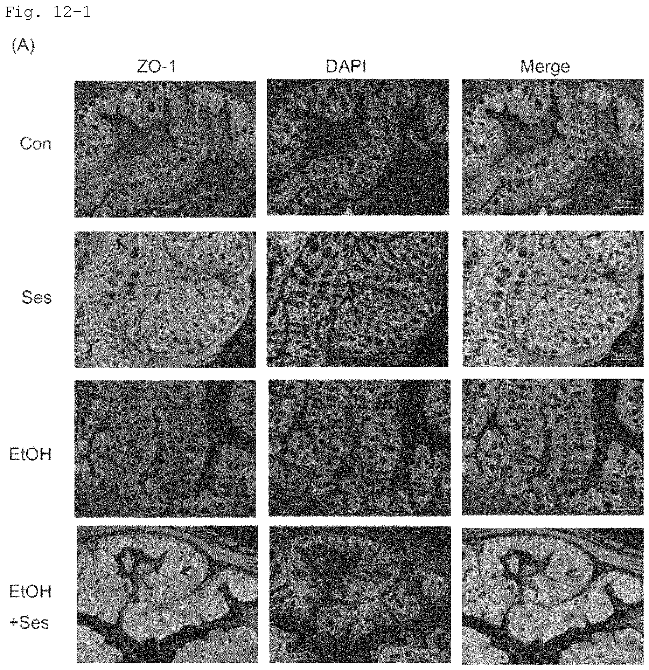

Fig. 12-1 shows the results of histological analysis of colonic epithelial TJ proteins (ZO-1, Occludin, and Claudin-1). The colon tissue pieces of 4-week-old mice were observed under a microscope. (A): Immunofluorescence-stained images using anti-ZO-1 antibody.

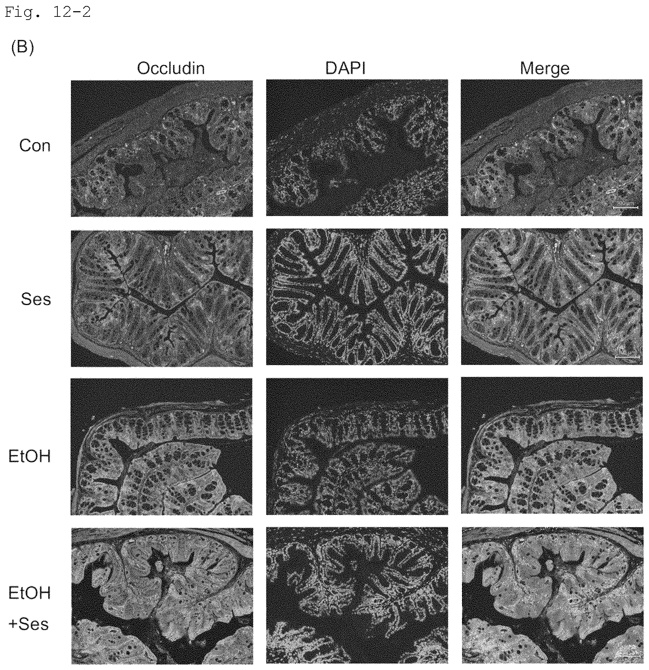

Fig. 12-2 shows the results of histological analysis of colonic epithelial TJ proteins (ZO-1, Occludin, and Claudin-1). The colon tissue pieces of 4-week-old mice were observed under a microscope. (B): Immunofluorescence-stained images using anti-Occludin antibody.

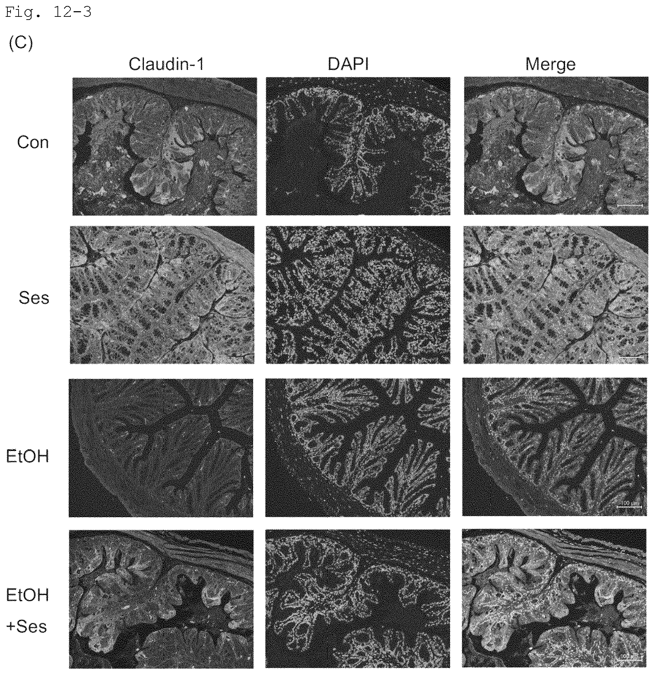

Fig. 12-3 shows the results of histological analysis of colonic epithelial TJ proteins (ZO-1, Occludin, and Claudin-1). The colon tissue pieces of 4-week-old mice were observed under a microscope. (C): Immunofluorescence-stained images using anti-Claudin-1 antibody.

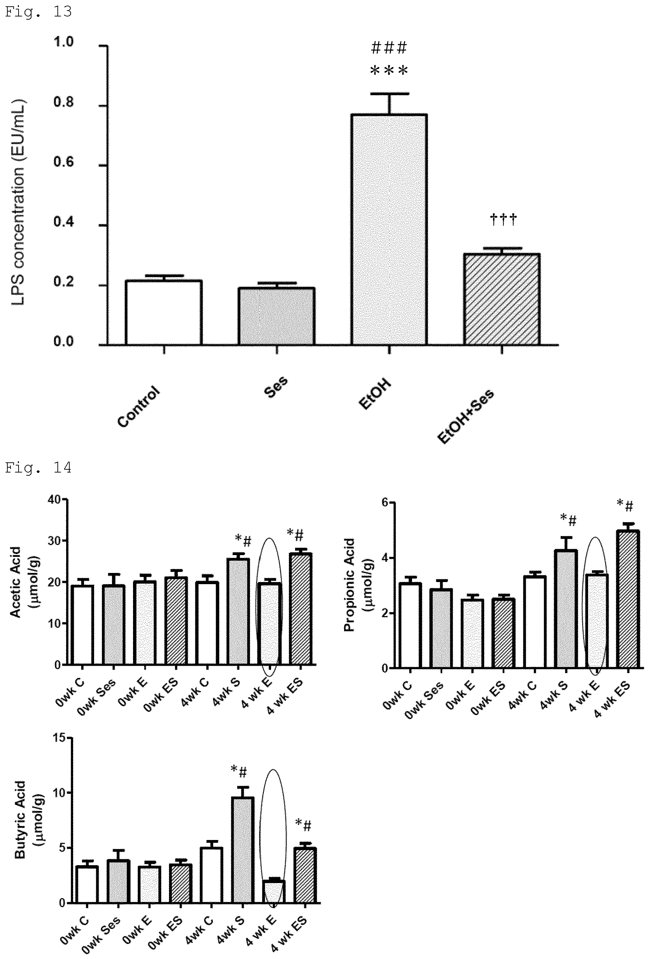

Fig. 13 shows the measurement results of LPS levels in mouse plasma. The data show the mean ±SD (n=8).
Fig. 14 shows the measurement results of the amounts of short-chain fatty acids (acetic acid, propionic acid, and butyric acid) in mouse feces. *: significant difference with respect to group C; ^{#}; significant difference with respect to group E.

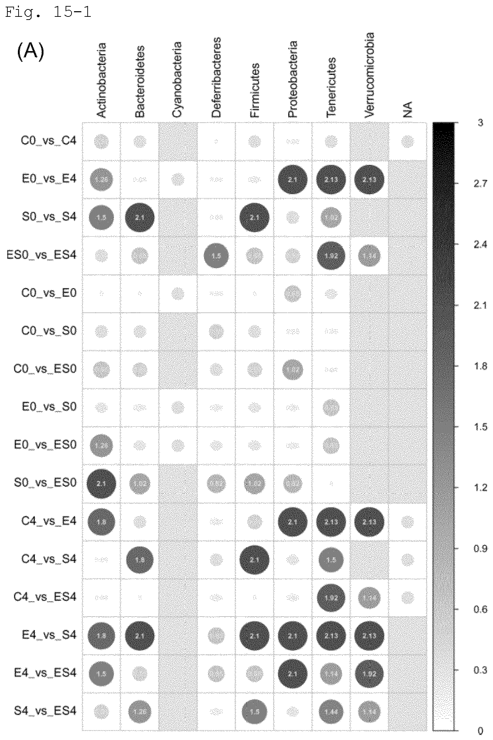

Fig. 15-1 shows the results of structural analysis of mouse gut microbiota. (A): Bacterial flora structural change level and evaluation for each group at phylum level.

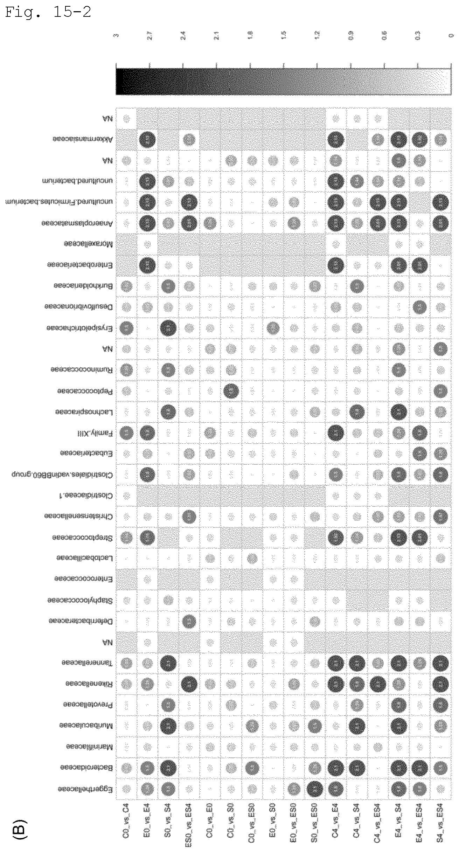

Fig. 15-2 shows the results of structural analysis of mouse gut microbiota. (B): Bacterial flora structural change level and evaluation for each group at family level.

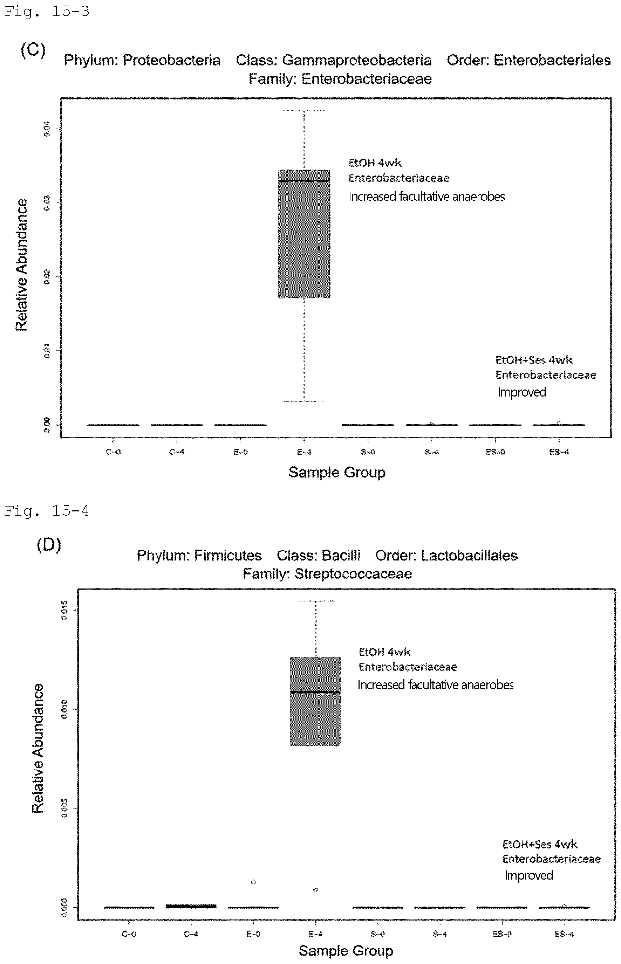

Fig. 15-3 shows the results of structural analysis of mouse gut microbiota. (C): Relative abundance of the family *Enterobacteriaceae.*
Fig. 15-4 shows the results of structural analysis of mouse gut microbiota. (D): Relative abundance of the family *Streptococcaceae.*

### Description of Embodiments

In the present specification, the expressions "contain," "comprising," and "include" include the concepts of containing, comprising, consisting essentially of, and consisting of.

In one embodiment, the present invention relates to a composition for suppressing enteritis, comprising at least one member selected from the group consisting of sesaminol and a metabolite thereof (which may be referred to herein as "the composition for suppressing enteritis of the present invention"). This composition is described below.

The composition for suppressing enteritis of the present invention comprises sesaminol or a metabolite thereof.

Sesaminol is a known compound having the following structural formula and is known to be found, for example, in sesame seeds.

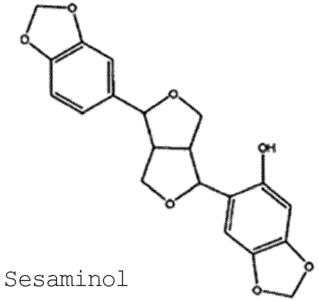

Research has been conducted on methods for efficiently producing sesaminol (see, for example, JP2006-61115A and JP2008-167712A); thus, sesaminol can be easily obtained or produced.

Examples of metabolites of sesaminol include both sesaminol metabolites in the blood and sesaminol metabolites in the digestive tract. Of these, metabolites in the digestive tract are preferred.

A report using rats has reported that orally administered sesaminol is absorbed in the small intestine and metabolized in the liver into 5''-methylated sesamin-6-catechol and sesaminol-6-catechol, and that those blood metabolites have, although it is for a short time, approximately 1.6 times more antioxidant activity than that of sesaminol (NPL 11).

Sesaminol metabolites in the digestive tract refer to those in which orally consumed sesaminol is metabolized in the digestive tract, such as the oral cavity, esophagus, stomach, small intestine (including duodenum, jejunum, and ileum), and large intestine (including cecum, colon, and rectum). Specific examples of sesaminol metabolites in the digestive tract include tetrahydrofuranoid (NPL 12).

The composition for suppressing enteritis of the present invention may consist of at least one member selected from the group consisting of sesaminol and a metabolite thereof, or may also comprise a component other than sesaminol and metabolites thereof as long as the effects of the present invention are not impaired. Specifically, the composition for suppressing enteritis of the present invention may be formed of 100 wt% of at least one member selected from the group consisting of sesaminol and a metabolite thereof, or may also comprise other components as long as the effects of the present invention are not impaired. The content of the other components is not particularly limited as long as the effects of the present invention are not impaired and can be, for example, 0.01 to 99.99 wt%, and preferably 0.1 to 99.9 wt%. Examples of the other components include pharmaceutically or food-hygienically acceptable bases, carriers, and additives. More specific examples include the components described below. The content of the at least one member selected from the group consisting of sesaminol and a metabolite thereof in the composition for suppressing enteritis of the present invention is, in particular, preferably 0.00001 wt% or more, more preferably 0.0001 wt% or more, still more preferably 0.001 wt% or more, and even more preferably 0.005 wt% or more. In one embodiment of the present invention, the upper limit of the content of the at least one member selected from the group consisting of sesaminol and a metabolite thereof in the composition for suppressing enteritis of the present invention is, for example, 100 wt%, and the lower limit is, for example, 0.01 wt%, 0.1 wt%, 1 wt%, 2 wt%, 5 wt%, 10 wt%, 20 wt%, 40 wt%, 60 wt%, or 80 wt%. The percentage of the content of the at least one member selected from the group consisting of sesaminol and a metabolite thereof per 100 wt% of the active ingredient for suppressing enteritis in the composition for suppressing enteritis of the present invention is, for example, 50 wt% or more, preferably 60 wt% or more, 70 wt% or more, 80 wt% or more, 90 wt% or more, 95 wt% or more, or 99 wt% or more.

The form of the composition of the present invention is not particularly limited and may be a commonly used form for each use depending on the use of the composition of the present invention.

Subjects to whom the composition for suppressing enteritis of the present invention is administered or fed are not particularly limited. Examples include healthy humans and patients with enteritis. The composition for suppressing enteritis of the present invention can be administered or fed to healthy humans to prevent enteritis, and to patients with enteritis to prevent the worsening of enteritis or to treat enteritis. Among healthy humans, for those who have a higher risk of enteritis, such as those who consume a large amount of alcohol, the administration or feeding can be performed more preferably for prevention. For administration or feeding to patients with enteritis, the cause of enteritis is not particularly limited. Examples of enteritis include chronic enteritis, acute enteritis, and drug-induced enteritis. Examples of chronic enteritis include infectious enteritis, alcoholic enteritis, intractable inflammatory bowel diseases, and enteritis caused by collagen disease. Examples of infectious enteritis include bacterial enteritis, parasitic enteritis, and viral enteritis. Examples of intractable inflammatory bowel diseases include ulcerative colitis and Crohn's disease. Examples of acute enteritis include infectious enteritis and ischemic enteritis. Ischemic colitis is often found in patients with lifestyle-related diseases and the elderly. Examples of drug-induced enteritis include pseudomembranous enteritis, drug-resistant bacterial enteritis (e.g., MRSA), and antibiotic-induced acute hemorrhagic enteritis, which cause antibiotic-induced bacterial turnover, resistant bacteria, and bleeding by non-steroidal anti-inflammatory drugs. Among these, chronic enteritis is preferred.

The subjects of administration/feeding of the composition for suppressing enteritis of the present invention include not only humans but also healthy animals or animals with symptoms of enteritis (in particular, pets and livestock). Examples of animals include dogs, cats, monkeys, mice, rats, hamsters, cows, horses, pigs, and sheep.

The route of the administration of the composition for suppressing enteritis of the present invention is preferably, in particular, oral administration or administration by injection (e.g., subcutaneous administration, intramuscular administration, transvenous administration, and transarterial administration).

The amount of administration or feeding of the prevention or therapeutic agent for enteritis of the present invention may be set appropriately. As a reference, the amount of sesaminol in the agent may be preferably in the range of 1 to 1,000 mg, and more preferably 10 to 500 mg, per adult per day. The administration or feeding can be performed once or divided multiple times (for example, preferably two to three times) per day.

One of the characteristics of the present invention is to suppress intestinal tissue damage by administration or feeding of at least one member selected from the group consisting of sesaminol and a metabolite thereof. Suppressing intestinal tissue damage means, for example, that death, dysfunction, proliferation inhibition, or the like is prevented from being induced in the cells constituting intestinal mucosa, intestinal submucosa, etc. Accordingly, the present invention encompasses a composition for suppressing intestinal tissue damage, comprising a member selected from the group consisting of sesaminol and a metabolite thereof.

One of the characteristics of the present invention is to protect intestinal mucosa by administration or feeding of sesaminol or a metabolite thereof. Protecting intestinal mucosa means, for example, suppressing damage to the mucosa of intestinal tissue or means preventing mucus reduction by preventing the reduction in the number of mucus-secreting goblet cells. Accordingly, the present invention encompasses a composition for protecting intestinal mucosa, comprising a member selected from the group consisting of sesaminol and a metabolite thereof.

One of the characteristics of the present invention is to suppress oxidative stress in intestinal tissue by administration or feeding of sesaminol or a metabolite thereof. Suppressing oxidative stress in intestinal tissue means, for example, suppressing the increase in the expression levels of oxidative stress-related genes or proteins encoded by those genes in intestinal tissue, or means maintaining these expression levels at about the same levels as those in normal intestinal tissue. Accordingly, the present invention encompasses a composition for suppressing oxidative stress in intestinal tissue, comprising a member selected from the group consisting of sesaminol and a metabolite thereof.

One of the characteristics of the present invention is to suppress the symptoms of leaky gut syndrome by administration or feeding of sesaminol or a metabolite thereof. Suppressing the symptoms of leaky gut syndrome means, for example, suppressing the decrease in the expression levels of membrane proteins that make up tight junctions, which are intercellular adhesion structures localized to epithelial cells in intestinal tissue, or means maintaining them at about the same levels as the expression levels thereof in normal intestinal tissue. Accordingly, the present invention encompasses a composition for suppressing leaky gut syndrome, comprising a member selected from the group consisting of sesaminol and a metabolite thereof.

One of the characteristics of the present invention is to improve gut microbiota by administration or feeding of sesaminol or a metabolite thereof. For example, improving gut microbiota means increasing or suppressing a decrease of the relative abundance of short-chain fatty acids-producing bacteria in gut microbiota compared with the relative abundance thereof in normal gut microbiota, or maintaining it closer to the relative abundance thereof in normal gut microbiota, or means improving gut microbiota imbalance, i.e., dysbiosis, to a closer state of normal gut microbiota. Accordingly, the present invention encompasses a composition for improving gut microbiota, comprising a member selected from the group consisting of sesaminol and a metabolite thereof.

Short-chain fatty acids are not particularly limited as long as they are fatty acids with less than 6 carbon atoms. Examples include acetic acid, propionic acid, and butyric acid. Short-chain fatty acids-producing bacteria are not particularly limited as long as they produce fatty acids with less than 6 carbon atoms. Preferably, short-chain fatty acids-producing bacteria are belonging to the phylum *Bacteroidetes* and the phylum *Firmicutes.* Therefore, improving gut microbiota more specifically means increasing or suppressing a decrease of the relative abundance of, for example, at least one short-chain fatty acids-producing bacteria selected from the group consisting of the phylum *Bacteroidetes* and the phylum *Firmicutes* in gut microbiota compared with the relative abundance thereof in normal gut microbiota, or means maintaining it closer to the relative abundance thereof in normal gut microbiota. Examples of bacteria belonging to the phylum *Bacteroidetes* include those from the family *Tannerellaceae* and the like. In particular, the family *Tannerellaceae* is preferred. Examples of bacteria belonging to the phylum *Firmicutes* include the family *Streptococcaceae,* the family *Lachnospiraceae,* including the genus *Blautia* and the genus *Roseburia,* and the family *Ruminococcaceae,* including the genus *Butyricicoccus,* the genus *Negativibacillus,* the genus *Oscillibacter,* and the genus *Ruminiclostridium.* In particular, the family *Lachnospiraceae* and the family *Ruminococcaceae* are preferred.

Gut microbiota imbalance is caused by, for example, alcohol consumption. Therefore, improving gut microbiota more specifically means, for example, suppressing an increase of the relative abundance of intestinal bacteria that increase with alcohol consumption in gut microbiota or maintaining it closer to the relative abundance thereof in normal gut microbiota, or means suppressing a decrease of the relative abundance of intestinal bacteria that decrease with alcohol consumption in gut microbiota or maintaining it closer to the relative abundance thereof in normal gut microbiota.

Examples of intestinal bacteria that increase with alcohol consumption include intestinal bacteria belonging to the phylum *Tenericutes,* the phylum *Verrucomicrobia,* the phylum *Proteobacteria,* the phylum *Actinobacteria,* and the phylum *Firmicutes.* Preferably, the intestinal bacteria that increase with alcohol consumption are intestinal bacteria belonging to the phylum *Tenericutes,* the phylum *Verrucomicrobia,* the phylum *Proteobacteria,* the phylum *Actinobacteria,* and the phylum *Firmicutes.* Therefore, improving gut microbiota more specifically means suppressing an increase of the relative abundance of, for example, at least one intestinal bacterium that increases with alcohol consumption and that is selected from the group consisting of the phylum *Tenericutes,* the phylum *Verrucomicrobia,* the phylum *Proteobacteria,* the phylum *Actinobacteria,* and the phylum *Firmicutes* in gut microbiota, or means maintaining it closer to the relative abundance thereof in normal gut microbiota. The intestinal bacteria of the phylum *Proteobacteria* are preferably those of the family *Enterobacteriaceae.* The intestinal bacteria of the phylum *Firmicutes* are preferably those of the family *Streptococcaceae.*

Examples of intestinal bacteria that decrease with alcohol consumption include intestinal bacteria belonging to the phylum *Bacteroidetes* and the phylum *Firmicutes.* Preferably, the intestinal bacteria that decrease with alcohol consumption are intestinal bacteria that belong to the phylum *Bacteroidetes* and the phylum *Firmicutes.* Therefore, improving gut microbiota more specifically means suppressing the alcohol-consumption-induced decrease of the relative abundance of, for example, at least one intestinal bacterium that is selected from the group consisting of the phylum *Bacteroidetes* and the phylum *Firmicutes* in gut microbiota or maintaining it closer to the relative abundance thereof in normal gut microbiota. The intestinal bacteria of the phylum *Bacteroidetes* are preferably those of the family *Tannerellaceae,* and the intestinal bacteria of the phylum *Firmicutes* are preferably those of the family *Lachnospiraceae* and the family *Ruminococcaceae.*

The above characteristics (suppression of intestinal tissue damage, protection of intestinal mucosa, suppression of oxidative stress in intestinal tissue, suppression of leaky gut syndrome, and improvement of gut microbiota) directly or indirectly act on suppressing enteritis. Further, suppressing enteritis directly or indirectly act on the above characteristics (suppression of intestinal tissue damage, protection of intestinal mucosa, suppression of oxidative stress in intestinal tissue, suppression of leaky gut syndrome, and improvement of gut microbiota).

The constituent components of the composition for suppressing intestinal tissue damage, the composition for suppressing oxidative stress in intestinal tissue, the composition for suppressing oxidative stress in intestinal tissue, the composition for suppressing leaky gut syndrome, and the composition for improving gut microbiota are the same as those of the composition for suppressing enteritis of the present invention.

The composition for suppressing enteritis of the present invention, the composition for suppressing intestinal tissue damage, the composition for suppressing oxidative stress in intestinal tissue, the composition for suppressing oxidative stress in intestinal tissue, the composition for suppressing leaky gut syndrome, and the composition for improving gut microbiota described above (these may be collectively referred to below as "the composition of the present invention") may be preferably used in the fields of pharmaceuticals and foods.

When the composition of the present invention is used in the field of pharmaceuticals, the composition of the present invention may be sesaminol itself, or may comprise sesaminol and optionally other pharmacologically active components, pharmaceutically acceptable bases, carriers, additives (e.g., solvents, dispersants, emulsifiers, buffers, stabilizers, excipients, binders, disintegrants, and lubricants) and the like. For example, the composition of the present invention may be prepared into pharmaceutical preparations, such as tablets, pills, powders, solutions, suspensions, emulsions, granules, capsules, injections, and drips. The preparation above can be performed according to common methods. Such a composition of the present invention can be preferably used for prevention or treatment of enteritis conditions, in particular, by oral administration, as mentioned above. That is, the composition of the present invention can be preferably used, for example, as an oral agent etc. In terms of the composition of the present invention, the conditions, such as the daily consumption amount of sesaminol, the subject for consumption, the content of sesaminol, and the content of other components, are preferably the same as those described above.

When the composition of the present invention is used as a food additive, the composition of the present invention may be sesaminol itself, or may comprise sesaminol in appropriate combination with food-hygienically acceptable bases, carriers, and additives, as well as other components, materials, etc. that can be used as food additives. Examples of the form of the food additive include, but are not limited to, liquid, powder, flake, granule, and paste forms. Specific examples include seasonings (e.g., soy sauce, sauce, ketchup, and dressing), flakes (*furikake*), grilled meat sauce, spices, and roux paste (e.g., curry roux paste). The food additive can be appropriately prepared according to common methods.

The food additive above according to the present invention is consumed by eating food to which the food additive is added. The addition may be performed during cooking or producing food, or may be performed immediately before or while eating cooked food. By oral consumption in this manner, the food additive exerts the effect of preventing enteritis. In terms of the food additive according to the present invention, the conditions, such as the daily consumption amount of sesaminol, the subject for consumption, the content of sesaminol, and the content of other components, are preferably the same as those described above.

When the composition of the present invention is used as a food and drink product for preventing enteritis, the composition (which is sometimes referred to below as "the food and drink product according to the present invention") comprises sesaminol in appropriate combination with food-hygienically acceptable bases, carriers, and additives, as well as other components, materials, etc. that can be used as food products. Such a composition of the present invention can also be referred to as "the food and drink product for preventing enteritis, comprising sesaminol." Examples include processed foods, drinks, health foods (e.g., foods with nutrient function claims and foods for specified health uses), supplements, and medical foods (e.g., hospital foods, foods for the sick, and nursing care foods), all of which comprise sesaminol and are for preventing enteritis. Examples of specific forms thereof include tablet, capsule, and drink preparation forms.

When the food and drink product according to the present invention is prepared as health foods (e.g., foods with nutrient function claims and foods for specified health uses) or supplements, the form is preferably, for example, granules, capsules, tablets (including chewable agents etc.), and drinks (drink preparations) to facilitate continuous consumption. Among these, the form is preferably, but is not particularly limited to, capsules, tablets, or pills from the viewpoint of ease of consumption. An agent for preventing enteritis in the form of granules, capsules, tablets, etc., comprising this food and drink product can be suitably prepared according to common methods using pharmaceutically and/or food-hygienically acceptable carriers and the like. The preparation into other forms can also be performed according to common methods.

In terms of the food and drink product according to the present invention, the consumption amount of sesaminol, the subject for consumption, the content of sesaminol, the content of other components, etc. are preferably, for example, the same those described above.

The present invention also provides a method for suppressing, improving, or treating enteritis, comprising administering or feeding the composition of the present invention to a patient with enteritis. The present invention also provides a method for preventing enteritis, comprising administering or feeding the composition of the present invention to a non-enteritis patient (i.e., a patient without enteritis, including healthy humans and those with borderline enteritis). These methods are specifically performed by administering or feeding the composition of the present invention described above. In these methods, the conditions, including the consumption amount of sesaminol contained in the composition of the present invention, are as described above.

The present invention also encompasses a method for suppressing intestinal cell damage in a subject (including a patient with enteritis and a non-enteritis patient), the method comprising orally administering or orally feeding sesaminol or a composition comprising sesaminol to the subject (with the proviso that a therapeutic method is excluded). The present invention also encompasses a method for improving the intestinal cell survival rate when death of intestinal cells in the subject occurs due to a specific factor (e.g., the factors described above) by orally administering or orally feeding sesaminol or a composition comprising sesaminol to the subject (with the proviso that a therapeutic method is excluded).

The statement "with the proviso that a therapeutic method is excluded" in these methods has the meaning of excluding actions performed based on the supervision, guidance, techniques, etc. of medical professionals (particularly doctors), and by extension, has the meaning of eliminating situations in which medical professionals have to perform medical procedures while fearing that what they are attempting to do may incur liability for patent infringement.

### Examples

The present invention is described in more detail below based on Examples. However, the present invention is not limited to these Examples. Below, "%" indicates mass%, unless otherwise specified.

Sesaminol used in the following Examples was prepared according to the method described in JP2008-167712A. More specifically, sesaminol was prepared according to the following preparation example.

### Preparation Example

*Paenibacillus* sp. KB0549 strain (deposit number: FERM P-21057) was cultured with defatted sesame debris (produced by Takemoto Oil & Fat Co., Ltd.), and sesaminol was produced from sesaminol glycosides contained in the defatted sesame debris. The details are described as follows.

First, KB0549 was grown in a warm-water extract of defatted sesame debris with 1.0% tryptone, 0.5% yeast extract, and 0.89% NaCl to obtain a KB0549 culture solution. The culture solution was added to 10.0 kg of defatted sesame debris (which had been heat-sterilized with the water content being adjusted to 70% and the pH to 6.0), and the mixture was fermented in a solid fermenter (37°C) with intermittent stirring and aeration continuously for 6 days.

Next, the fermented defatted sesame debris thus obtained was dried to a water content of 8.5%. Subsequently, 95% ethanol in a volume 10 times the weight of the dried product was added, and the mixture was heated to 50°C and stirred to extract sesaminol. The resulting extract was filtered through a filter press with diatomaceous earth to remove solids to obtain 82 L of a filtrate. The filtrate (82 L) was concentrated to 4.1 L in a vacuum concentrator. Then, 99.5% ethanol in an amount at least 4 times that of the resulting concentrated liquid was added, and insoluble matter was removed by filtration with filter paper, followed by concentration in an evaporator to obtain 4.05 L of a highly concentrated liquid.

The sesaminol and sesaminol-related compounds in the highly concentrated liquid were identified by high-performance liquid chromatography (HPLC), the results of which indicated that the concentrated liquid contained 18.4 g of sesaminol. The HPLC analysis conditions are as follows:
HPLC: Hitachi LaChrom
Column: Wakosil-II 5C18HG (4.6 mm (diameter) × 250 mm, Wako Pure Chemical Industries, Ltd.)
Developing solvent:
   A: 10% acetonitrile + 0.1% trifluoroacetic acid
   B: 80% acetonitrile + 0.1% trifluoroacetic acid
   Development of a linear gradient of 10-100% B (40 min).
Flow rate: 0.8 ml/min
Analytical wavelength: 280 nm

The sesaminol and sesaminol-related compounds in the highly concentrated liquid were identified and the amounts thereof were calculated by preparing a calibration curve using standard samples of sesaminol and sesaminol-related compounds. The sesaminol content can be further increased by additional use of solvent extraction, silica gel column purification, gel filtration purification, and other methods.

### Example 1: Change in Body Weight by Administration of Sesaminol and General Observation

Ten-week-old C57BL/6NCr male mice were divided into four groups (eight mice per group), i.e., group C, group S, group E, and group ES, and fed daily for 2 weeks in the following manner. The mice in group C as the control group were fed a liquid diet containing neither ethanol nor sesaminol (dextrose-controlled liquid diet). The mice in group S were force-fed a liquid diet containing 2.5 mg sesaminol per day (equivalent to 100 mg kg⁻¹ d⁻¹) by oral administration. The mice in group E and group ES were freely fed an ethanol-containing liquid diet (2.0% v/v ethanol) with the amount of energy adjusted to be equal to that of the control group. The mice in group ES were force-fed a liquid diet containing 2.5 mg of sesaminol per day by oral administration.

The body weights of the mice in the above four groups (group C, group S, group E, and group ES) were measured every week. No significant differences were observed in body-weight changes between the groups during the test period (Fig. 1). All of the mice used in the experiment did not die suddenly during the test period and also did not suffer from loss of appetite or immobility.

### Example 2: Effect of Oral Administration of Sesaminol on Ethanol-induced Lesion in Mouse Colon

The colonic epithelium of the mice in groups C, E, S, and ES were stained with hematoxylin-eosin (HE) and toluidine blue (TB), and observed under a microscope (Figs. 2(A) and (B)). Immunostaining was then performed by using rabbit anti-mucin 2 (MUC2) polyclonal antibody (Fig. 2(C)).

### HE Staining and TB Staining of Tissue Specimen

HE-stained and TB-stained tissue specimens were prepared according to the following procedure. Specifically, a portion of colon tissue was collected, fixed in a 10% v/v neutral formaldehyde solution, and paraffin-embedded. Thereafter, paraffin sections (4-um-thick) were prepared and stained with HE and TB.

### Immunostaining of Tissue Specimen

Immunostained tissue specimens were prepared according to the following procedure.

The paraffin sections above were placed on glass slides, heated at 60°C for 2 hours to dry, deparaffinized by washing three times with xylene for 5 minutes, and then dehydrated with ethanol. For heat-induced antigen recovery, the glass slides were heated at 100°C for 20 minutes in 10 mM sodium citrate buffer (pH of 6.0), cooled to room temperature in the same buffer for 20 minutes, and washed with 1x Tris-buffered saline containing 0.1% Tween 20. The sections were incubated with 3% H₂O₂ for 5 minutes to inhibit endogenous peroxidase activity. The sections were incubated for 30 minutes at room temperature with 2% bovine serum albumin in phosphate-buffered saline and then incubated overnight at 4°C with rabbit anti-MUC2 polyclonal antibody. The sections were then incubated with biotinylated goat anti-rabbit IgG antibody for 20 minutes at room temperature, visualized by peroxidase-catalyzed oxidation of diaminobenzene, and contrast-stained with hematoxylin.

Fig. 2 shows the results. No obvious lesions were observed in group C and group S. A structural change was observed such that the mucosal layer in the colonic epithelium of the mice in group E was thinner than that in the colonic epithelium in group C (Figs. 2(A) and (B)). Further, the colon tissue of the mice in group E showed a decrease in the amount of mucus secretion due to a decrease in the number of goblet cells (Figs. 2(B) and (C)). On the other hand, the colonic epithelium of the mice in group ES showed a smaller degree of lesion compared with that observed in group E (Figs. 2(A) to (C)). A comparison of the scores of microscopic observation of the colon tissue (colon epithelium, colon crypts, submucosa, and mucosa) between the groups resulted in the highest score of group E (Fig. 2(D)). The score of group ES was significantly lower than that of group E, although it was higher than those of group C and group S, which did not show any lesions (Fig. 2(D)).

### Example 3: Effect of Oral Administration of Sesaminol on Ethanol-induced Oxidative Stress in Mouse Colon

The present inventors have previously reported that chronic oral administration of ethanol to mice increases oxidative stress in colon tissue (NPL 13). Therefore, in order to verify the effect of oral administration of sesaminol on oxidative stress in the colon induced by chronic oral consumption of ethanol, the levels of oxidative stress markers (8-hydroxy-2'-deoxyguanosine (8-OHdG), malondialdehyde (MDA), and 4-hydroxyalkenals (HAE)) were examined in colon tissue of the mice in groups C, S, E, and ES.

The level of 8-OHdG, which is an oxidative stress marker in tissue, was examined by immunohistochemical staining and ELISA.

### Immunohistochemical Staining

Immunostained tissue specimens were prepared in the same manner as the method described above, except that the primary antibody was rabbit anti-8-OHdG monocolonal antibody.

### ELISA

A colon tissue sample (50 mg) was homogenized in 1 mL of DNAzol reagent, and the resulting homogenate was centrifuged at 20,000× g at 4°C for 3 hours. DNA was then isolated from the supernatant by ethanol precipitation and treated with an 8-OHdG assay preparation reagent set (FUJIFILM Wako Pure Chemical Corporation). Equal amounts of the extracted DNA were used for analysis of the 8-OHdG level using an OxiSelect oxidative DNA damage ELISA kit (Cell Biolabs, Inc.).

Fig. 3(A) shows the results. As a result of immunohistochemical staining, no appreciable level of 8-OHdG was detected in the mice in group C and group S (Fig. 3(A)). In contrast, the mice in group E showed the highest 8-OHdG level in colon tissue, and the mice in group ES showed the next highest level (Fig. 3(A)). Fig. 3(B) shows the results of quantitative measurement by ELISA.

Lipid peroxidation is used as an indicator of oxidative stress in cells and tissues; lipid peroxides produce MDA and HAE upon degradation. Therefore, the MDA+HAE level in colon tissue was examined as a marker of lipid peroxidation response.

### Lipid Peroxide Measurement

A colon tissue sample (100 mg) was homogenized in 1 mL of 20 mM phosphate buffered saline (pH of 7.4) with a homogenizer and centrifuged at 3,000× g at 4°C for 10 minutes. The resulting precipitate was examined for malondialdehyde (MDA) and 4-hydroxyalkenals (HAE) using a lipid peroxide assay kit (Oxford Biomedical Research, Inc.).

Fig. 3(C) shows the results. Group E showed the highest MDA+HAE level in colon tissue, and group ES showed the next highest value (Fig. 3(C)). The value of group ES was slightly higher than those of group C and group S (Fig. 3(C)).

### Example 4: Effect of Oral Administration of Sesaminol on Expression of Ethanol-induced Oxidative Stress Protein and Transcription Factor Protein in Mouse Colon

Chronic consumption of an excessive amount of ethanol has been reported to increase CYP2E1 and iNOS levels and cause ethanol-induced oxidative stress in the liver and other tissues. The nuclear factor erythroid 2-related factor 2/heme oxygenase-1 (Nrf2/HO-1) system has also been reported to be widely involved in the protection of cells from oxidative damage. Therefore, the expression levels of these proteins (CYP2E1, iNOS, and HO-1) in mouse colon tissue in the four groups (groups C, S, E, and ES) were examined by western blotting (WB).

### Western Blotting

A colon tissue sample (100 mg) was homogenized in 1 mL of an M-PER mammalian protein extraction reagent (Pierce) containing a diluent of a protease inhibitor cocktail (Nacalai Tesque, Inc.), and the resulting homogenate was centrifuged at 20,000× g at 4°C for 15 minutes. The protein concentration of the supernatant was measured with a protein assay reagent (Bio-Rad Laboratories, Inc.), and equal amounts of proteins were subjected to SDS-PAGE. Electrophoresed proteins in the gel were transferred to a PVDF membrane, and the membrane was immersed in a blocking reagent (room temperature, overnight). The primary antibody (rabbit polyclonal antibody) was then reacted. The following primary antibodies were used.
Anti-mouse CYP2E1
Anti-mouse inducible nitric oxide synthase (iNOS)
Anti-mouse heme oxygenase-1 (HO-1)
Anti-mouse β-Actin

After the primary antibody reaction, washing was performed three times with 1× Tris buffered saline containing 0.1% Tween 20 for 10 minutes. The resulting product was reacted with HRP-conjugated anti-rabbit IgG, and visualized by chemiluminescence reaction catalyzed by peroxidase using an ECL Western blotting kit (Cytiva). The band intensities were quantified with ImageJ.

Fig. 4 shows the results. The CYP2E1 expression level in group E was significantly higher than those in group C and the other groups. The CYP2E1 expression level in group ES was significantly lower than that in group E (Figs. 4(A) and (B)). Similar results were also observed in the iNOS expression level (Figs. 4(A) and (B)). The HO-1 expression level in group E was significantly lower than that in group C; however, group ES showed a recovery of the expression level (Figs. 4(A) and (B)).

The Nrf2 transcription factor is a master regulator of cytoprotection and promotes HO-1 gene expression in intracellular response to oxidative stress. Thus, in terms of the decrease in the HO-1 expression level in group E, the activation level of the Nrf2 transcription factor was examined. The protein amounts in the colon tissue extracts of the groups were adjusted to be equal, and Nrf2 activation levels in the colon tissue extracts were determined by ELISA.

Fig. 5(C) shows the results. The Nrf2 activation level was significantly reduced in group E compared with group C; however, the level in group ES recovered to a level similar to that in group C (Fig. 4(C)).

### Example 5: Effect of Oral Administration of Sesaminol on Ethanol-induced colitis in Mouse Large Intestine

It has been reported that chronic consumption of an excessive amount of ethanol can induce inflammation associated with tumor formation in colon tissue. Therefore, in order to verify whether oral administration of sesaminol has a preventive effect on ethanol-induced colitis, the expression levels of inflammatory cytokines (TNF-α and IL-6) and chemokine (MCP-1) in the large intestinal lamina propria of the mice in the four groups (groups C, S, E, and ES) were examined by qRT-PCR and ELISA. The NF-κB activity in the mice in the four groups was also examined. NF-κB is a transcription factor that is involved in expression of genes regulating inflammation and is activated by exposure of immune cells to pro-inflammatory factors.

### qRT-PCR

Total RNA was extracted from colon tissue samples. The qRT-PCR conditions were as follows.
95°C for 10 seconds
60°C for 10 seconds
72°C for 10-20 seconds
45 cycles

### ELISA

A colon tissue sample (100 mg) was homogenized in 1 mL of an M-PER mammalian protein extraction reagent (Pierce) containing a diluent of a protease inhibitor cocktail (Nacalai Tesque, Inc.), and the resulting homogenate was centrifuged at 20,000× g at 4°C for 15 minutes. The protein concentration of the supernatant was measured with a protein assay reagent (Bio-Rad Laboratories, Inc.), and equal amounts of proteins were used for analysis of the expression levels of TNF-α, IL-6, and MCP-1 by

### ELISA.

Fig. 5 shows the results. The expression of TNF-α, IL-6, and MCP-1 genes in colon tissue of the mice were examined by qRT-PCR (Figs. 5(A) to (C)), and the expression of the proteins were examined by ELISA (Figs. 5(D) to (F)). The results indicated that the expression levels of these genes in group E were all significantly higher than those in group C. The expression levels of TNF-α, IL-6, and MCP-1 genes in group ES were higher than those in group C but were significantly lower than those in group E (Figs. 5(A) to (F)). Similarly, although the NF-κB p65 activation level in group E was significantly higher than that in group C, the NF-κB p65 activation level in group ES was significantly lower than that in group E (Fig. 5(G)).

### Example 6: Effect of Oral Administration of Sesaminol on Ethanol-induced Intestinal Tight Junction Damage and Leaky Gut

Acetaldehyde, which accumulates during ethanol metabolism, is known to disrupt intestinal tight junctions (TJs) via dissociation of colonic epithelium TJ proteins (ZO-1, occludin, and claudin-1) from the cytoskeleton. This results in loss of intercellular adhesion and increased permeability of bacterial toxins (lipopolysaccharides (LPS)) from the colon lumen into the body circulation, potentially causing endotoxemia. Therefore, whether oral administration of sesaminol would affect ethanol-induced intestinal barrier dysfunction was examined by analyzing the expression levels of colonic epithelial TJ proteins and the plasma LPS level in the mice in the four groups (groups C, S, E, and ES) .

### Western Blotting

WB was performed in the same manner as in Example 4 above. The following primary antibodies were used.
Anti-mouse zonula occludens-1 protein (ZO-1) rabbit polyclonal antibody
Anti-mouse occludin rabbit polyclonal antibody
Anti-mouse claudin-1 rabbit polyclonal antibody

### Measurement of Plasma LPS Level

The plasma LPS concentration was measured with a limulus amebocyte lysate (LAL) assay.

Fig. 6 shows the results. The expression levels of ZO-1, occludin, and claudin-1 in colon tissue of the mice in group E were significantly decreased compared with those in group C (Figs. 6(A) and (B)). The expression levels of ZO-1, occludin, and claudin-1 in group ES were lower than those in group C but were significantly higher than those in group E. Further, the plasma LPS level of the mice in group E was significantly higher than that in group C. The LPS level in group ES was also higher than that in group C but was significantly lower than that in group E (Fig. 6(C)).

These results confirmed that oral administration of sesaminol suppresses ethanol-induced symptoms (tissue lesions, oxidative stress production, inflammation, and leaky gut due to intestinal TJ damage) (Fig. 7).

### Example 7: Change in Body Weight by Administration of Sesaminol and General Observation

Ten-week-old C57BL/6NCr male mice were divided into four groups (eight mice per group), i.e., group C, group S, group E, and group ES, and fed daily for 4 weeks in the same manner as in Example 1.

The body weights of the mice in the above four groups (group C, group S, group E, and group ES) were measured every week. No significant differences were observed in body-weight changes between the groups during the test period (Fig. 8). All of the mice used in the experiment did not die suddenly during the test period and also did not suffer from loss of appetite or immobility.

### Example 8: Effect of Oral Administration of Sesaminol on Ethanol-induced Lesion in Mouse Liver

The liver function and plasma lipid level in the mice in each group were examined by measuring the percentage of the liver weight relative to the body weight, the triglyceride (TG) concentration in the liver, the aspartate aminotransferase (AST) activity in the plasma, the alanine aminotransferase (ALT) activity in the plasma, and the TG concentration in the plasma of the mice in each group.

### Measurement of Percentage of Liver Weight Relative to Body Weight

The body weight of mice fed for 4 weeks were weighed and dissected under anesthesia. The liver was removed and weighed. The weight ratio between the body weight and the liver of each individual was evaluated.

### Measurement of TG Amount in the Liver

Each liver was homogenized at 4°C, and TG was extracted according to the product protocol of a reagent kit for TG extraction from tissue (Lipid Droplet Isolation Kit, produced by Cell Biolabs, Inc.). The amounts of TG and protein in each extracted sample were quantified according to the product protocol of each measuring reagent and converted to the TG amount (mg) per protein (g) for evaluation.

Measurement of AST and ALT Activities and TG Concentration in the Plasma

The AST and ALT activities and TG concentration in the plasma were measured according to the product protocol using dedicated reagent kits for each test item (L-Type Wako AST, L-Type Wako ALT, and L-Type Wako TG M, produced by FUJIFILM Wako Pure Chemical Corporation).

Table 1 shows the results.

**Table 1**

| Value | Con | Sesaminol (2.5 mg/d) | EtOH (2.0% v/v) | EtOH +Sesaminol |
|---|---|---|---|---|
| Body weight (g) | 26.3±0.6 | 25.9±0.8 | 25.7±0.5 | 25.4±1.0 |
| Liver/body weight ratio (g/g, %) | 3.98±0.30 | 3.96±0.55 | 4.68±0.49*^{, †} | 4.04±0.41^{#} |
| Liver TG (mg/g of protein) | 34.5±6.8 | 38.1±12.4 | 82.3±19.9***^{, †††} | 51.8±9.5^{###} |
| Plasma AST (U/L) | 37.6±8.0 | 41.3±6.8 | 57.4±9.2***^{, ††} | 43.5±7.6^{##} |
| Plasma ALT (U/L) | 31.4±6.5 | 33.3±5.7 | 86.3±12.9***^{, †††} | 45.8±6.6*^{, †, ###} |
| Plasma TG (mmol/L) | 0.55±0.16 | 0.53±0.14 | 1.01±0.26***^{, †††} | 0.65±0.13^{##} |

| | | | | |
|---|---|---|---|---|
| ALT: Alanine aminotransferase, TG: triglycerides, AST: aspartate aminotransferase, ALT: alanine aminotransferase. Each value is expressed as "mean ±SD." Comparisons between groups were evaluated using ANOVA and Tukey-Kramer test. *: p<0.05, ***: p<0.001 vs. Con (C) group, †: p<0.05, ^{††}: p<0.01, and ^{†††}: p<0.001 vs. Sesaminol (S) group, ^{#}: p<0.05, ^{##}: p<0.01, ^{###}: p<0.001 vs. EtOH (E) group. EtOH+Sesaminol (ES) group. | | | | |

The weight ratio of the liver to the body weight increased in group E compared with that of group C, but decreased in group ES compared with that of group E. Further, the TG concentrations in the liver and plasma increased in group E compared with those in group C but decreased in group ES compared with those in group E. Furthermore, plasma AST and ALT activities increased in group E compared with those in group C but decreased in group ES compared with those in group E.

Additionally, liver sections of the mice in each group (groups C, E, S, and ES) were stained with HE or Oil Red O, and observed under a microscope.

### HE Staining or Oil Red O Staining of Liver Sections

Liver tissue samples were each fixed in a 10% v/v neutral formaldehyde solution overnight at room temperature and paraffin-embedded. The liver tissue sections were then stained with HE reagent, and frozen tissue sections were stained with Oil Red O reagent. Each stained specimen was observed under a microscope and subjected to image analysis.

Fig. 9 shows the results. The microscopic images of the Oil Red O-stained liver tissue showed a significant increase in lipid droplets stained with red in group E but showed a decrease in lipid droplets in group ES. These observation results indicate that oral administration of sesaminol improves alcoholic steatohepatitis (Fig. 9(B)).

### Example 9: Effect of Oral Administration of Sesaminol on Ethanol-induced Oxidative Stress in Mouse Colon

In order to verify the longer-term effect of oral administration of sesaminol on ethanol-induced oxidative stress in mouse colon, the colonic epithelium of the mice in each group (groups C, E, S, and ES) described in Example 7 was HE-stained, TB-stained, and periodic-acid-Schiff (PAS)-stained and observed under a microscope (Figs. 10(A), (B), and (C)). Immunostaining with MUC2 polyclonal antibody was then performed (Fig. 10(D)). HE staining, TB staining, and immunostaining with MUC2 polyclonal antibody were performed in the same manner as in Example 2.

### PAS Staining of Colon Tissue Specimen

Colon tissue samples were each fixed in a 10% v/v neutral formaldehyde solution overnight at room temperature and paraffin-embedded. The colon tissue sections were then stained with PAS staining reagent for the detection of goblet cells. Each stained specimen was observed under a microscope and subjected to image analysis.

Fig. 10 shows the results. The results of observation of HE-stained, TB-stained, PAS-stained, or MUC2-immunostained colon tissue revealed that atrophy of colonic epithelium tissue and a significant decrease in goblet cells and mucus-secreting cells were observed in group E compared with group C; however, in group ES, the colon tissue that had been damaged by continuous ethanol administration showed a tendency of returning to the histological image of group C.

These results indicated that oral administration of sesaminol improves inflammation of colon caused by continuous alcohol administration.

### Example 10: Effect of Oral Administration of Sesaminol on the Amount of Mucus Secretion in Mouse Colon

The effect of oral administration of sesaminol on the amount of mucus secretion in mouse colon was examined by measuring the amount of fecal mucin of the mice in each group (groups C, E, S, and ES) and the amount of secretory immunoglobulin A (sIgA) in the colon tissue of the mice.

### Measurement of Amount of Fecal Mucin of Mice

Fecal samples were each freeze-dried overnight in a freeze-drying equipment and then formed into a powder. After weighing each of the dried, powdered feces, fecal mucin was quantified according to the product protocol of a reagent kit for measuring fecal mucin (Fecal Mucin Assay Kit, produced by Cosmo Bio Co., Ltd.), and converted to the mucin amount (µg) per amount of dry feces (g). The results were evaluated.

### Measurement of Amount of Secretory IgA in Colon Tissue of Mice

After liquid nitrogen treatment, the amount of sIgA in each colon tissue stored at -80°C was quantified according to the product protocol of a reagent kit for measuring secretory IgA (QuickDetect (trademark) Secretory IgA (Mouse) ELISA kit, produced by BioVision, Inc.). The resulting value was converted to the secretory IgA concentration (ng/mL) per 100 mg of the tissue and evaluated.

Fig. 11 shows the results. The amount of fecal mucin of the mice in group E showed a significant and considerable decrease compared with that of group C. On the other hand, the amount of fecal mucin of the mice in group ES was significantly higher than that in group E, showing an increase to a level similar to that in group C (Fig. 11(A)). Further, the amount of secretory IgA in the mouse colon tissue in group E showed a significant and considerable decrease compared with that of group C. On the other hand, the amount of secretory IgA in the mouse colon tissue in group ES was significantly higher than that in group E, showing an increase to a level similar to that in group C (Fig. 11(B)). These results indicated that oral administration of sesaminol suppresses both the decrease in the amount of colonic mucus secretion and the decrease in the amount of secretory IgA caused by continuous ethanol administration.

### Example 11: Preventive Effect of Sesaminol on Ethanol-Induced Intestinal Tight Junction Damage and Leaky Gut

Next, in order to verify the longer-term effect of oral administration of sesaminol on ethanol-induced intestinal tight junction (TJ) dysfunction and leaky gut, histological analysis of colonic epithelial TJ proteins (ZO-1, Occludin, and Claudin-1) and analysis of the plasma LPS level were performed using the mice in each group (groups C, S, E, and ES) described in Example 7. Histological analysis was performed by immunofluorescence staining with anti-ZO-1, anti-Occludin, and anti-Claudin-1 antibodies, followed by observation under a fluorescence microscope. The Plasma LPS level was measured according to the method described in Example 6.

Fig. 12 shows the results of fluorescence microscope observation, and Fig. 13 shows the results of the measurement of plasma LPS level.

Figs. 12(A) to (C) show fluorescence microscope observation of protein levels of ZO-1, Occludin, and Claudin-1, which are tight junction (cell-to-cell junction) markers, in colon tissue. It was observed that group E showed a significant decrease in ZO-1, Occludin, and Claudin-1 compared with group C and group S. On the other hand, group ES showed an improvement in all of those levels.

In Fig. 13, group E showed a significant and considerable increase in the plasma LPS concentration compared with group C; however, group ES showed a significant and considerable decrease in the plasma LPS concentration compared with group E and showed an improvement to a level similar to those in group C and group S. These results indicated that oral administration of sesaminol has a long-term preventive effect on ethanol-induced colonic TJ dysfunction and leaky gut (decreased intestinal barrier function).

### Example 12: Effect of Oral Administration of Sesaminol on Gut Microbiota Structure in Mice

Short-chain fatty acids are metabolites of intestinal bacteria. The amounts of short-chain fatty acids (acetic acid, propionic acid, and butyric acid) in feces of the mice in each group (groups C, E, S, and ES) were measured.

### Measurement of Amount of Short-chain Fatty Acids in Mouse Feces

The amounts of short-chain fatty acids in the mouse feces were measured by outsourcing to TechnoSuruga Laboratory Co., Ltd.

Immediately after collection, mouse feces was treated with liquid nitrogen and stored at -80°C. While the fecal samples were frozen, an amount of 100 mg was crushed with beads and formed into a powder, and components extracted with ethyl acetate treatment were used as detection samples. For the measurement, a gas-chromatography-flame ionization detector system (Agilent 7890B GC, produced by Agilent technologies) was used, and the concentration of each short-chain fatty acid was calculated by comparison with the amount of standard substance.

Fig. 14 shows the results. Continuous administration of ethanol and sesaminol to each group for 4 weeks resulted in a significant and considerable decrease in the amount of butyric acid in the feces in group E compared with all the other groups. Group ES showed a significant improvement, showing an increase in the amount of butyric acid to the same level as that in group C. On the other hand, there was no difference between group C and group E in terms of the amounts of acetic acid and propionic acid in the feces.

As a characteristic trend, group S showed a significant increase in the amount of short-chain fatty acids in the feces compared with all the other groups, including group C. Group ES also showed a significant increase in acetic acid and propionic acid in the feces.

These results indicated that oral administration of sesaminol suppresses for a long time an ethanol-induced reduction of butyric acid in feces. The results also indicated that administration of sesaminol alone increases three types of short-chain fatty acids (acetic acid, propionic acid, and butyric acid) in feces.

Additionally, the gut microbiota structures of the mice in each group (groups C, E, S, and ES) were analyzed.

Structural Analysis of Mouse Gut Microbiota

The mouse gut microbiota 16S rRNA sequencing was outsourced to MyMetagenome Co., Ltd.

Immediately after collection, mouse feces was treated with liquid nitrogen and stored at -80°C. While the fecal samples were frozen, treatment was performed, and the extracted nucleic acids were used as samples for sequence analysis. The measurement was performed using a next-generation sequencer (NGS) (MiniSeq, Illumina, K.K.), and the results of 16S rRNA genes (the V1-V2 region) and sequences of 5,000 reads or more from the extracted nucleic acids were used for analysis. For the analysis of bacterial flora structure, raw sequence data processing and well-known sequence data analysis were performed, and the analysis was clustered at a 96% pairwise identity cutoff for 16S reads. Similarity values were assigned to the phylum and genus categories.

Fig. 15 shows the results.

Fig. 15(A) shows the results of gut microbiota structural analysis at the Phylum level. When group C and group E were compared, group E showed an increase in two phyla, the phylum *Tenericutes* and the phylum *Verrucomicrobia,* which are hardly detected in healthy conditions. Further, a significant increase in the phylum *Proteobacteria,* which is typically classified as a facultative anaerobe, was observed in group E compared with group C, and additionally an increase in the phylum *Actinobacteria* was observed in group E. In group ES, in which both ethanol and sesaminol were administered continuously, the significant abnormalities in the bacterial flora structure observed in group E were recovered to the same level as group C, indicating an improvement in the bacterial flora structure. As a characteristic phenomenon, an increase in obligate anaerobes of the phylum *Bacteroidetes* and the phylum *Firmicutes,* which are short-chain fatty acids-producing bacteria, was observed in group S alone compared with group C.

Fig. 15(B) shows the results of gut microbiota structural analysis at the family level. When group C and group E were compared, the relative abundance of the family *Enterobacteriaceae* and the family *Streptococcaceae,* which are facultative anaerobes, was significantly abnormal in group E, showing a significant increase (Fig. 15(C) and Fig. 15(D)). On the other hand, in group ES, the relative abundance of the family *Enterobacteriaceae* and the family *Streptococcaceae* in gut microbiota observed in group E recovered to the same level as that in group C, indicating a significant improvement. Further, a comparison between group C and group E revealed that the relative abundance of the family *Lachnospiraceae,* the family *Ruminococcaceae,* and the family *Tannerellaceae,* which are known as butyrate-producing bacteria, was significantly abnormal in group E, showing a significant decrease. On the other hand, in group ES, the abnormalities of these four families observed in group E recovered to the same level as that in group C, showing significant improvement. As a characteristic phenomenon, short-chain fatty acid-producing bacteria of the family *Lachnospiraceae,* the family *Ruminococcaceae,* and the family *Tannerellaceae* increased in group S alone compared with group C.

These results demonstrated that continuous oral consumption of sesaminol under conditions of continuous ethanol consumption prevented and improved gut microbiota structural abnormalities. The above results are closely associated with an increase in the relative abundance of butyric acid-producing bacteria, in addition to the results of the amounts of short-chain fatty acids in feces. Additionally, administration of sesaminol alone increased obligate anaerobes of the phylum *Bacteroidetes* and the phylum *Firmicutes,* which are short-chain fatty acids-producing bacteria, and also increased the family *Lachnospiraceae,* the family *Ruminococcaceae,* and the family *Tannerellaceae.*

## Claims

1. A composition for suppressing enteritis, comprising at least one member selected from the group consisting of sesaminol and a metabolite thereof.

2. The composition for suppressing enteritis according to claim 1, wherein the enteritis is chronic enteritis.

3. The composition for suppressing enteritis according to claim 1 or 2, for use in suppressing leaky gut syndrome.

4. The composition for suppressing enteritis according to any one of claims 1 to 3, which is an oral composition.

5. The composition for suppressing enteritis according to any one of claims 1 to 4, which is a food composition.

6. The composition for suppressing enteritis according to any one of claims 1 to 5, wherein the metabolite is a sesaminol metabolite in the digestive tract.

7. A composition for suppressing intestinal tissue damage, comprising at least one member selected from the group consisting of sesaminol and a metabolite thereof.

8. A composition for protecting intestinal mucosa, comprising at least one member selected from the group consisting of sesaminol and a metabolite thereof.

9. A composition for suppressing oxidative stress in intestinal tissue, comprising at least one member selected from the group consisting of sesaminol and a metabolite thereof.

10. A composition for suppressing leaky gut syndrome, comprising at least one member selected from the group consisting of sesaminol and a metabolite thereof.

11. A composition for improving gut microbiota, comprising at least one member selected from the group consisting of sesaminol and a metabolite thereof.

12. A method for suppressing enteritis, comprising administering or feeding at least one member selected from the group consisting of sesaminol and a metabolite thereof to a subject in need of suppressing enteritis.

13. A composition comprising at least one member selected from the group consisting of sesaminol and a metabolite thereof, for use as a composition for suppressing enteritis.

14. Use of a composition comprising at least one member selected from the group consisting of sesaminol and a metabolite thereof, in the production of a composition for suppressing enteritis.

15. Use of a composition comprising at least one member selected from the group consisting of sesaminol and a metabolite thereof, as a composition for suppressing enteritis.
